# FASCICULE DE BREVET EUROPEEN

(11) **EP 0 613 470 B1**
(45) Date de publication et mention de la délivrance du brevet: **10.01.1996**
(21) Numéro de dépôt: 92924754.2
(22) Date de dépôt: 03.11.1992
(51) Int. Cl.: C07D 237/28

(54) **NOUVEAUX DERIVES DE CINNOLINONES-4, LEUR PREPARATION ET LEUR APPLICATION EN THERAPEUTIQUE**
4-CINNOLINON-DERIVATE, IHRE HERSTELLUNG UND IHRE VERWENDUNG ALS ARZNEIMITTEL
NOVEL 4-CINNOLINONE DERIVATIVES, THEIR PREPARATION AND APPLICATION IN THERAPY

(30) Priorité: 04.11.1991 FR 9113571
(43) Date de publication de la demande: 07.09.1994
(73) Titulaire: PIERRE FABRE MEDICAMENT, F-92654 Boulogne Cédex (FR)
(72) Inventeur: PATOISEAU, Jean-François, F-81100 Castres (FR); AUTIN, Jean-Marie, F-81290 Labrugière (FR); MAUREL, Jean-Louis, F-81570 Vielmur s/Agout (FR); BIGG, Dennis, F-81100 Castres (FR)
(74) Mandataire: Ahner, Francis
(86) Numéro de dépôt international: FR9201019
(87) Numéro de publication internationale: WO9309098

(56) Documents cités:
- EP-A- 0 277 791
- US-A- 3 937 704
- JOURNAL OF THE CHEMICAL SOCIETY (C) 1971, LETCHWORTH GB pages 3088 - 3097 D.E. AMES ET AL 'Cinnolines. Part XV. Methylation of Methoxy- and Alkyl-cinnolines and -4(1H)-cinnolines.'

## Description

La présente invention, réalisée au Centre de Recherche PIERRE FABRE MEDICAMENT, a pour objet de nouveaux composés chimiques, leur procédé de préparation et leur application en tant que médicaments.

Des cinnolinones-4 diversement fonctionnalisées en position 3 sont reportées dans la littérature.
Ainsi, les acides carboxyliques et certains de leurs esters possèdent des propriétés antibactériennes (demandes de brevets US n° 796546 et 888.880) ou antiallergiques (demandes de brevets GB n° 49282/70 et 38483/71). Les amides présentent des propriétés anxiolytiques ou immunomodulatrices (brevet EP 277.791 et demande de brevet GB n° 13639/85). La présence d'une fonction méthylsulfinyl ou méthylsulfonyl en 3 confère une activité immunostimulante (brevet US n° 3,937.704).
La synthèse de tels composés a été décrite ainsi que différentes méthodes générales d'accès aux cinnolinones-4.

La présente invention a plus particulièrement pour objet les composés de formule générale I : 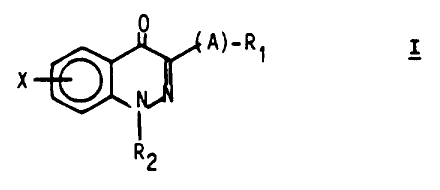 dans laquelle :
- A représente l'oxygène ou un groupement NR₃ dans lequel R₃ représente lui-même l'hydrogène ou un radical alcoyle C₁₋₄.
- R₁ représente :
   . l'hydrogène ou un radical alcoyle C₁₋₄
   . un radical phénylalcoyle C₁₋₄, le noyau aromatique pouvant être substitué par un ou plusieurs groupements tel que halogène, alcoyle C₁₋₃, alcoyl C₁₋₃ oxy, trifluorométhyle,
   . un groupement aminoalcoyle 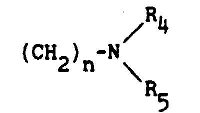 dans lequel n représente un entier compris entre 1 et 4 inclus et R₄, R₅ identiques ou différents représentent l'hydrogène, un radical alcoyle C₁₋₃ ou forment ensemble avec l'atome d'azote auquel ils sont liés un hétérocycle tel que pyrrolidine, pipéridine, morpholine ou pipérazine.
- R₂ représente :
   . un radical alcoyl C₂₋₅, alcenyl C₃₋₅, hydroxyalcoyle C₂₋₅, alcoxy C₁₋₃ alcoyle C₁₋₄
   . un radical phényle ou phényl alcoyle C₁₋₃, le noyau aromatique pouvant être substitué par un ou plusieurs groupements tels que halogène, alcoyle C₁₋₃, alcoyl C₁₋₃ oxy, trifluorométhyle.
- X représente un ou plusieurs groupements tel que hydrogène, halogène, alcoyle C₁₋₃, alcoyl C₁₋₃ oxy, trifluorométhyl.

La présente invention a également pour objet un procédé de préparation des composés de formule générale I, caractérisé en ce que :
- Soit l'on traite un acide dihydro-1,4 cinnolinone-4 carboxylique-3 de formule générale II 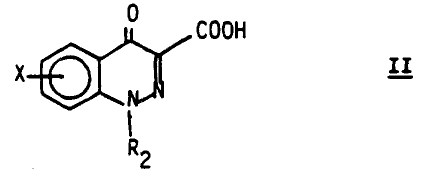 par le diphénylphosphoryl azide en présence d'un alcool R₅OH, à température ambiante puis chauffage à une température comprise entre 50°C et la température d'ébullition de l'alcool pour former, via l'isocyanate, les composés de formule générale III 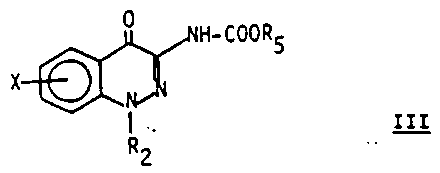 R₂ et X ont la même signification que précédemment et R₅ représente un radical alcoyle C₁₋₅ linéaire ou ramifié. L'alcool peut notamment être le tertiobutanol. Les composés III sont traités optionnellement par un dérivé R₃Y, dans lequel R₃ est différent de H, ou un dialcoyl C₁₋₂ sulfate (composés IVb) puis soumis à une hydrolyse acide pour accéder aux composés de formule générale IVa ou IVb 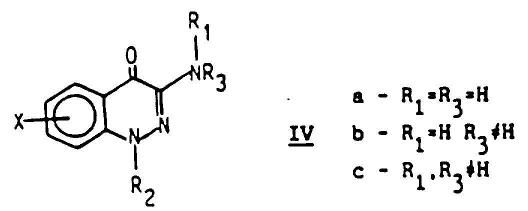 R₁, R₂, R₃, X ont la même signification que précédemment et Y représente un groupement labile tel qu'halogène ou mésylate.
   Les acides cinnolinone-4 carboxyliques-3 de formule II peuvent être obtenus selon les méthodes décrites telles que :
   D.E. AMES et coll. J. Chem. Soc., 1964, p. 5659-5663
   D.E. AMES et coll. Synthesis, 1983, p. 52-53.
- Soit l'on traite une dihydro-1,4 méthyl sulfonyl-3 cinnolinone-4 de formule générale V 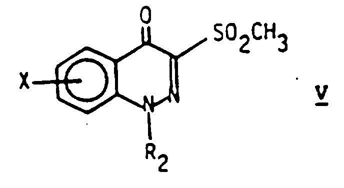 X et R₂ ayant la même signification que précédemment, par le formamide dans le diméthylformamide en présence d'une base forte telle que l'hydrure de sodium ou de potassium, puis par la soude ou la potasse alcoolique, notamment éthanolique, pour donner les composés de structure IVa tels que définis ci-dessus.

Les composés de structure générale V sont accessibles selon les méthodes décrites, notamment le brevet US n° 3,937.704.

Les composés de formule générale IVa ou IVb peuvent être alcoylés selon les méthodes classiques utilisées pour alcoyler les amines tel que, à titre d'exemple, traitement par un halogénure d'alcoyle.

Les composés de formule générale IVa peuvent également être traités :
. soit par le formaldéhyde dans l'acide formique puis au cyanoborohydrure de sodium pour conduire aux composés diméthylés correspondants IVc avec R₁=R₃=CH₃ ;
. soit par un nitrite d'alcoyle tel que le nitrite de tertiobutyle en présence de chlorure cuivreux anhydre dans un milieu anhydre tel que, à titre d'exemple, le DMF en présence de tamis moléculaires pour accéder aux composés de formule VI 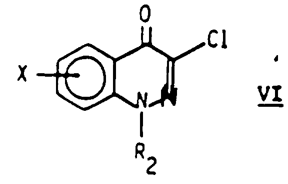 dans laquelle R₂ et X ont la même signification que précédemment.

Les composés de formule VI traités par une amine VII 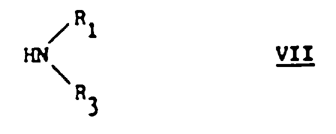 conduisent aux composés de formule générale IVc ;
. soit par le nitrite de sodium en milieu chlorhydrique pour accéder aux dérivés hydroxylés de formule générale VIIIa 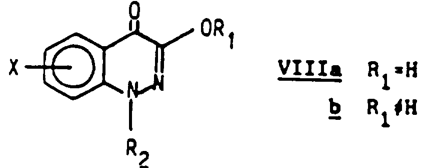 dans laquelle R₁, R₂ et X ont la même signification que précédemment.

Les composés de formule générale VIIIa, traités par un dérivé R₁Y avec R₁≠ H et Y étant tel que défini précédemment, fournissent les composés VIIIb.
La réaction peut être effectuée en présence d'une base forte telle que soude, potasse, hydrure de sodium ou de potassium dans un solvant tel que DMF, DMSO ou THF.
Elle peut également être avantageusement conduite en milieu biphasique, constitué d'une phase aqueuse telle que soude ou potasse et organique telle que THF ou éther éthylique, en présence d'un catalyseur de transfert de phase tel que, à titre d'exemple, un sel d'ammonium quaternaire comme le chlorure ou le bromure de tétrabutylammonium.

Les exemples ci-après illustrent la présente invention. Les analyses centésimales ainsi que les spectres IR et RMN confirment la structure des composés obtenus.

### Exemple 1

### Amino-3 dihydro-1,4 éthyl-1 cinnolinone-4 1

A une suspension d'acide dihydro-1,4 éthyl-1 cinnolinone-4 carboxylique-3 (1,05 g, 4,8 mmoles) dans le tertiobutanol (10 ml), on ajoute 0,7 ml de triéthylamine et 2,64 g (9,6 mmoles) de diphényl phosphorylazide puis chauffe le mélange 6 H à reflux. Le solvant est évaporé sous vide et le résidu repris par une solution de bicarbonate de sodium et extrait à l'acétate d'éthyle. Après lavage à l'eau, séchage sur sulfate de sodium et évaporation sous vide, on obtient une huile (carbamate de tertiobutyle intermédiaire) qui est reprise par l'acide trifluoroacétique (10 ml). Après agitation 1 H à température ambiante, et évaporation sous vide, le résidu est repris au bicarbonate de sodium et extrait au chlorure de méthylène. Après lavage à l'eau, séchage sur sulfate de sodium et évaporation sous vide, on obtient par cristallisation dans l'éther éthylique 0,63 g de composé 1 (Rendement = 68 %) F = 182°C 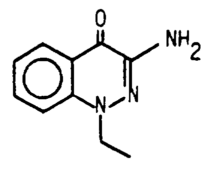 RMN (CDCl₃) : 1,38 ppm, t, 3H ; 3.62 ppm, massif échangeable ; 4,26 ppm, q, 2H ; 7,13 ppm, dt, 1H; 7,22 ppm, d, 1H ; 7,51 ppm, dt, 1H ; 8,22 ppm, dd, 1H.

### Exemple 2

### Dihydro-1,4 métatrifluorométhylphényl-1 méthylamino-3 cinnolinone-4 2

La métatrifluorométhylphényl-1 dihydro-1,4 t.butyloxycarbonylamino-3 cinnolinone-4 (1 g, 2,46 mmoles) obtenue à partir de l'acide correspondant par traitement au DPPA dans le tertiobutanol de façon similaire à l'exemple 1, est mise en suspension dans le THF (30 ml). On ajoute 50 mg de CBTA, 1,5 ml de soude 6N et 0,3 ml d'iodure de méthyle. Après 6 H à température ambiante, le mélange réactionnel est additionné d'eau et extrait à l'acétate d'éthyle. La phase organique séchée sur Na₂SO₄ et évaporée sous vide fournit une huile épaisse qui est reprise par l'acide trifluoroacétique (5 ml). Après agitation 1 H à température ambiante, et évaporation sous vide, le résidu est repris au bicarbonate de sodium et filtré. Après lavage à l'eau, le produit est repris à l'acétate d'éthyle et filtré sur gel de silice (30 g) pour donner 0,68 g de composé 2 (Rendement = 87 %) F = 225°C 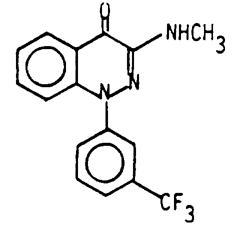 CCM : Rf = 0,8 (acétate d'éthyle-hexane 50-50)
RMN (CDCl₃) : 2,86 ppm, s, 3H ; 5,06 ppm, massif échangeable, 1H ; 7,02 à 7,73 ppm, massif complexe, 7H ; 8,20 ppm, dd, 1H.

### Exemple 3

### Amino-3 chloro-6 éthyl-1 dihydro-1,4 cinnolinone-4 3

A une suspension d'hydrure de sodium à 60 % (1,88 g, 47 mmoles) dans le DMF (60 ml), on ajoute goutte à goutte 7,8 ml de formamide. Dès la fin du dégagement d'hydrogène, on introduit l'éthyl-1 chloro-6 méthylsulfonyl-3 dihydro-1,4 cinnolinone-4 (9 g, 31 mmoles) et chauffe 2 H à 100°C sous agitation. Après évaporation sous vide du DMF, le mélange est repris par l'éthanol (100 ml) et la soude 6N (100 ml) et chauffé 1 H à 70°C. L'éthanol est évaporé, le résidu repris à l'eau et extrait au chloroforme (2 X 100 ml). La phase organique est lavée à l'eau, séchée et concentrée jusqu'a un volume de 30 ml. Purifier sur colonne de silice en éluant avec du chloroforme. Après évaporation à sec des différentes fractions, on obtient le composé 3 (3,56 g - Rendement = 51 %) F = 188°C 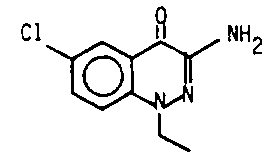 CCM : Rf = 0,25 (ACOEt-CHCl₃ 10-90)
RMN (CDCl₃) : 1,44 ppm, t, 3H ; 4,29 ppm, q, 2H ; 4,73 ppm, s échangeable, 2H ; 7,29 ppm, d, 1H ; 7,40 ppm, dd, 1H ; 8,24 ppm, d, 1H.

### Exemple 4

### Dihydro-1,4 diméthylamino-3 éthyl-1 cinnolinone-4 4

A une solution d'amino-3 dihydro-1,4 éthyl-1 cinnolinone-4 (exemple 1) (1,62 g, 8,3 mmoles) dans l'acide formique (55 ml), on ajoute du paraformaldéhyde (2,54 g ; 85 mmoles) puis du cyanoborohydrure de sodium (2,52 g, 40 mmoles). Après 18 H sous agitation à température ambiante, le mélange est jeté sur de la glace, neutralisé à la soude 6N puis extrait au chlorure de méthylène. Après lavage à l'eau, puis à l'eau salée, séchage sur Na₂SO₄ et évaporation sous vide, on obtient une huile jaune qui est purifiée par chromatographie flash. L'élution par un mélange acétate d'éthyle-hexane 30-70 fournit, après mise à sec, le composé 4 (1,02 g - Rendement = 48 %) F= 42°C 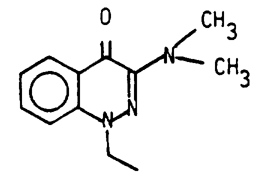 CCM : Rf = 0,5 (ACOEt-Hexane 30-70)
RMN (CDCl₃) : 1,46 ppm, t, 3H ; 3,04 ppm, s, 6H ; 4,35 ppm, q, 2H ; 7,19 ppm, t, 1H ; 7,32 ppm, d, 1H ; 7,58 ppm, dt, 1H ; 8,33 ppm, dd, 1H.

### Exemple 5

### Dihydro-1 ,4 métatrifluorométhylphényl-1 β morpholinoéthylamino-3 cinnolinone-4 5

a) A une solution de DMF maintenue à 0°C, on ajoute sucessivement des tamis moléculaires broyés (1,2 g) du nitrite de tertiobutyle (1,4 g) du chlorure cuivrique anhydre (0,82 g) et par petites portions l'amino-3 dihydro-1,4 métatrifluorométhylphényl-1 cinnolinone-4 (obtenue de façon analogue à l'exemple 1) (1,54 g) en solution dans le DMF (7 ml). Après agitation 30 mn à 0°C puis 1 H 30 à 60°C, le mélange est filtré puis évaporé sous vide. Le résidu est repris à l'eau, extrait à l'acétate d'éthyle.
   Après lavage à l'eau, séchage sur Na₂SO₄ évaporation à sec, puis purification sur colonne flash, on obtient la chloro-3 dihydro-1,4 métatrifluorométhylphényl-1 cinnolinone-4 (1 g - Rendement = 62 %) F = 199°C 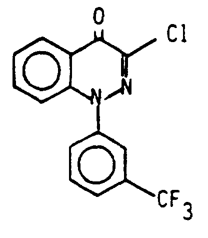 CCM : Rf = 0,7 (ACOEt-CHCI₃ 10-90)
b) Le composé chloré obtenu ci-dessus (0,98 g ; 3 mmoles) en solution dans l'(amino-2-éthyl)-4-morpholine (5,2 ml ; 20 mmoles) est chauffé pendant 36 H à 100°C.
   Après évaporation à sec sous vide, on obtient une huile jaune qui est reprise à l'eau et extraite à l'acétate d'éthyle.
   Après lavage à l'eau, séchage sur Na₂SO₄ et évaporation sous vide, on obtient un composé qui est purifié par chromatographie flash. Par élution à l'acétate d'éthyle, on obtient le composé 5 (0,45 g - Rendement = 36 %) F = 125°C 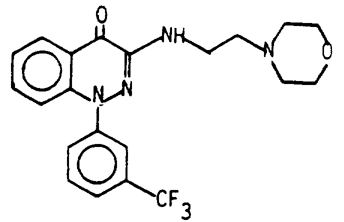 CCM : Rf = 0,38 (Acétate d'éthyle)
   RMN (CDCl₃) : 2,57 ppm, t, 4H ; 2,71 ppm, t, 2H ; 3,46 ppm, t, 2H ; 3,76 ppm, t, 4H ; 6,07 ppm, échangeable, 1H ; 7,10 à 7,80 ppm, massif complexe, 7H ; 8,31ppm, dd, 1H.

### Exemple 6

### Dihydro-1,4 éthyl-1 hydroxy-3 cinnolinone-4 6

Une suspension du composé 1 (exemple 1) (5,8 g ; 30 mmoles) dans l'eau (180 ml) est glacée. Après addition d'acide acétique (100 ml) et d'acide chlorhydrique 12N (5,5 ml), on ajoute goutte à goutte le nitrite de sodium (2,07 g ; 30 mmoles) dans l'eau (30 ml). Après agitation du mélange 1 H à 0°C puis 2 H à 60°C, on refroidit et extrait au dichlorométhane. Après lavage à l'eau, séchage sur Na₂SO₄ et concentration sous vide, on obtient le composé 6 qui cristallise par trituration dans l'éther éthylique
(3 g - Rendement = 53 %) F = 200°C 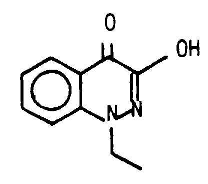 CCM : Rf = 0,6 (CHCl₃-MeOH 90-10)
RMN (DMSO) : 1,33 ppm, t, 3H ; 4,36 ppm, q, 2H ; 7,30 ppm, m 1H ; 7,72 ppm, d, 2H ; 8,12 ppm, d, 1H ; 10,76 ppm, s échangeable, 1H.

### Exemple 7

### Dihydro-1,4 éthyl-1 méthoxy-3 cinnolinone-4 7

Le composé 6 (1,90 g) en suspension dans le THF (100 ml) est additionné de soude 6N (6,6 ml) et le chlorure de tétrabutylammonium (0,322 g). On ajoute ensuite progressivement 2,83 ml de diméthylsulfate puis après 4 H d'agitation à température ambiante, évapore à sec sous vide. Le résidu est repris à l'eau et extrait à l'acétate d'éthyle. On obtient après lavage à l'eau, séchage sur Na₂SO₄, concentration sous vide puis purification par flash chromatographie (élution ACOEt-CHCl₃ 10-90), le composé 7 (1,22 g - Rendement = 60 %) F = 119° C 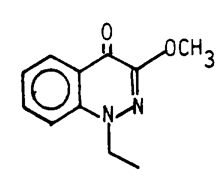 CCM : Rf = 0,45 (ACOEt-CHCl₃ 10-90)
RMN (CDCl₃) : 1,47 ppm, t, 3H ; 3,95 ppm, s, 3H ; 4,35 ppm, q, 2H ; 7,22 ppm, t, 1H ; 7,35 ppm. d, 1H ; 7,63 ppm, t, 1H ; 8,36 ppm, d, 1H.

Le tableau 1 ci-après résume les principaux produits synthétisés.

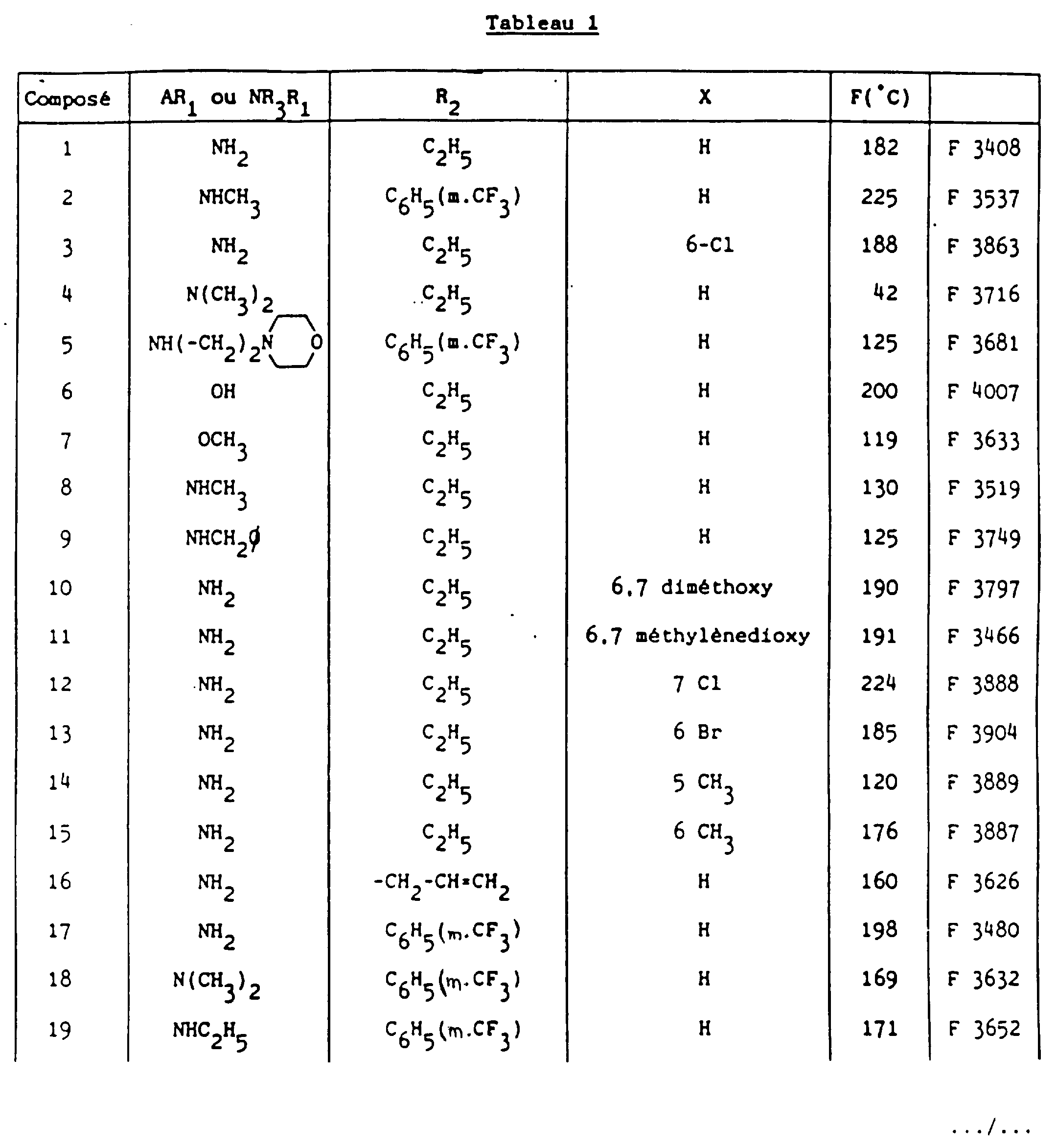

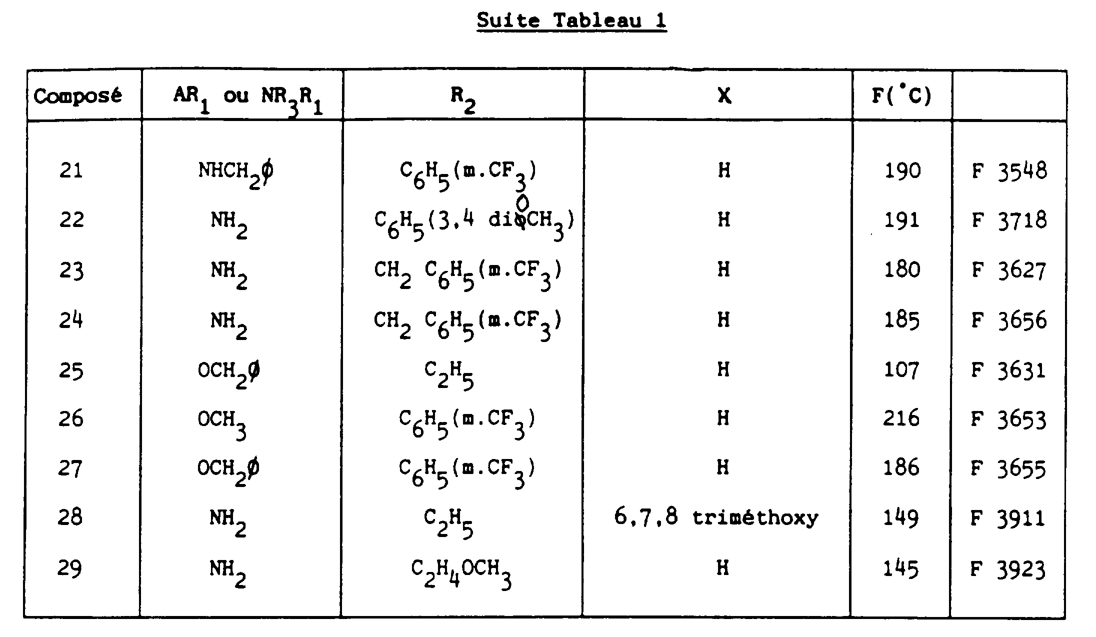

### EXPERIMENTATIONS BIOLOGIQUES

### 1 - Etude pharmacologique

Les composés de l'invention ont été soumis à des essais pharmacologiques qui ont montré leur intérêt en tant que broncholytiques.
A cet effet, les composés ont été étudiés pour leur effet antagoniste des contractions provoquées par divers produits selon le protocole suivant.

Un morceau de trachée de cobaye tricolore mâle de 400 g en moyenne est découpé en spirale et monté (2,5 cm environ) dans une cuve à organe isolé thermostatée à 37°C et remplie de tyrode oxygéné. On enregistre les contractions isométriques provoquées par les divers médiateurs grâce à un capteur UC2 Gould Statham (Gould Inc. Oxnard, California, USA) ou UF1 Palmer Bioscience relié à un enregistreur potentiométrique (Linseiss, Selb, RFA). Au début de l'expérience, le lambeau de trachée est soumis à une tension de 1 g et laissé au repos durant 1 H.
On utilise les concentrations suivantes de médiateurs (concentrations provoquant en général une contraction maximale): dichlorhydrate d'histamine, 10 µg/ml ; chlorure d'acétylcholine, 1 µg/ml ; sel de potassium de leucotriene D₄, 0,05 µg/ml ; chlorure de potassium, 1850 µg/ml. Les concentrations d'agents utilisés entraînent des contractions qui deviennent maximales au bout de 5 à 15 mn suivant les médiateurs et se maintiennent en palier ensuite. Une concentration du produit étudié est administrée pour chaque contraction et le produit est laissé au contact de la trachée durant 5 mn. On mesure l'inhibition éventuelle obtenue au bout de ce temps (pourcentage de variation de l'amplitude de la contraction). La trachée est ensuite lavée durant 15 secondes et laissée au repos durant 10 mn environ (temps nécessaire au retour au tonus de base) avant de provoquer une nouvelle contraction.

Les produits insolubles dans l'eau sont dilués dans 0,178 % (v/v) (concentration finale dans le bain) de diméthyl sulfoxide (DMSO). Cette concentration de DMSO n'entraîne aucun effet vis-à-vis des contractions des divers médiateurs étudiés.

Les concentrations inhibitrices 50 % (IC₅₀) sont calculées en utilisant un programme SAS (Statistical Analysis System) inspiré de Bliss et Cattel (BLISS C.I. et CATTEL McK. Biological Assay Ann. Rev. Physiol. 5, 479, 1943).

Les résultats obtenus sur certains composés de l'invention sont reportés, à titre d'exemple, dans les tableaux 2 à 5.

**Tableau 2**

| Antagonisme de l'effet du chlorure de potassium | |
|---|---|
| Composé n° | IC₅₀ (µg/ml) |
| 1 | 6 |
| 3 | 6,9 |
| 4 | 6,1 |
| 8 | 5,1 |
| 12 | 5,4 |
| 14 | 6,5 |
| 17 | 13 |
| 22 | 7,8 |
| Théophylline | 21 |

**Tableau 3**

| Antagonisme de l'effet de l'histamine | |
|---|---|
| Composé n° | IC₅₀ (µg/ml) |
| 1 | 5,3 |
| 3 | 7,1 |
| 4 | 2,2 |
| 8 | 2,1 |
| 12 | 3,4 |
| 14 | 9,5 |
| 17 | 11 |
| 22 | 8,7 |
| Théophylline | 11,5 |

**Tableau 4**

| Antagonisme de l'effet de l'acétylcholine | |
|---|---|
| Composé n° | IC₅₀ (µg/ml) |
| 1 | 9,4 |
| 3 | 22,1 |
| 4 | 19,9 |
| 8 | 18 |
| 12 | 7,9 |
| 14 | 20,7 |
| 17 | 23 |
| 22 | 10,3 |
| Théophylline | 65 |

**Tableau 5**

| Antagonisme de l'effet du leucotriene D₄ | |
|---|---|
| Composé n° | IC₅₀ (µg/ml) |
| 1 | - 55 % |
| 8 | - 88 % |
| Théophylline IC₅₀ = 13,5 µg/ml | |

### 2 - Applications thérapeutiques

Les composés de l'invention sont des broncholytiques qui peuvent être utilisés pour le traitement des maladies telles que les bronchopneumopathies obstructives chroniques, l'insuffisance respiratoire, l'emphysème, les pathologies cardiovasculaires relatives à l'insuffisance cardiaque.

Les compositions pharmaceutiques peuvent être sous forme appropriée pour l'administration par voie orale, rectale, parentérale ou locale, par exemple sous la forme de capsules, comprimés, granulés, gélules ou solutés liquides, sirops ou suspensions buvables, aérosols ou solutions pulvérisables, et contenir les excipients appropriés.

La posologie quotidienne peut aller de 50 à 1 000 mg.

## Revendications

1. Composés dérivés de cinnolinones-4 de formule générale I 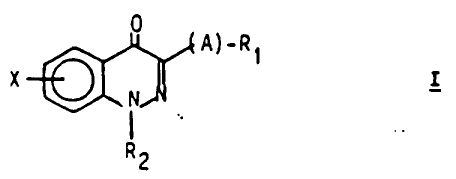 dans laquelle :
- A représente l'oxygène ou un groupement NR₃ dans lequel R₃ représente lui-même I'hydrogène ou un radical alcoyle C₁₋₄.
- R₁ représente :
. l'hydrogène ou un radical alcoyle C₁₋₄
. un radical phénylalcoyle C₁₋₄, le noyau aromatique pouvant être substitué par un ou plusieurs groupements choisis parmi : halogène, alcoyle C_{1-3,} alcoyl C₁₋₃ oxy, trifluorométhyle.
. un groupement aminoalcoyle 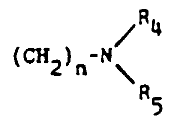 dans lequel n représente un entier compris entre 1 et 4 inclus et R₄, R₅ identiques ou différents représentent l'hydrogène, un radical alcoyle C₁₋₃ ou forment ensemble avec l'atome d'azote auquel ils sont liés un hétérocycle choisi parmi:pyrrolidine, pipéridine, morpholine ou pipérazine.
- R₂ représente :
. un radical alcoyl C_{2-5'} alcenyl C_{3-5'} hydroxyalcoyle C₂₋₅, alcoxy C₁₋₃ alcoyle C₁₋₄
. un radical phényle ou phényl alcoyle C_{1-3,} le noyau aromatique pouvant être substitué par un ou plusieurs groupements choisis parmi halogène, alcoyle C₁₋₃, alcoyl c₁₋₃ oxy, trifluorométhyle.
- X représente un ou plusieurs groupements choisis parmi hydrogène, halogène, alcoyle C₁₋₃, alcoyl C₁₋₃ oxy, trifluorométhyle.

2. Composés de formule générale I selon la revendication 1 caractérisés par le fait qu'ils sont choisis parmi :
- Amino-3 dihydro-1,4 éthyl-1 cinnolinone-4
- Dihydro-1,4 méthylamino-3 métatrifluorométhylphényl-1 cinnolinone-4
- Amino-3 chloro-6 éthyl-1 dihydro-1,4 cinnolinone-4
- Dihydro-1,4 diméthylamino-3 éthyl-1 cinnolinone-4
- Dihydro-1,4 métatrifluorométhylphényl-1 β morpholinoéthylamino-3 cinnolinone-4
- Dihydro-1,4 éthyl-1 hydroxy-3 cinnolinone-4
- Dihydro-1,4 éthyl-1 méthoxy-3 cinnolinone-4
- Dihydro-1,4 éthyl-1 méthylamino-3 cinnolinone-4
- Benzylamino-3 dihydro-1,4 éthyl-1 cinnolinone-4
- Amino-3 dihydro-1,4 diméthoxy-6,7 éthyl-1 cinnolinone-4
- Amino-3 dihydro-1,4 éthyl-1 méthylènedioxy-6,7 cinnolinone-4
- Amino-3 chloro-7 dihydro-1,4 éthyl-1 cinnolinone-4
- Amino-3 bromo-6 dihydro-1,4 éthyl-1 cinnolinone-4
- Amino-3 dihydro-1,4 éthyl-1 méthyl-5 cinnolinone-4
- Amino-3 dihydro-1,4 éthyl-1 méthyl-6 cinnolinone-4
- Allyl-1 amino-3 dihydro-1,4 cinnolinone-4
- Amino-3 dihydro-1,4 métatrifluorométhylphényl-1 cinnolinone-4
- Dihydro-1,4 diméthylamino-3 métatrifluorométhylphényl-1 cinnolinone-4
- Dihydro-1,4 éthylamino-3 métatrifluorométhylphényl-1 cinnolinone-4
- Benzylamino-3 dihydro-1,4 métatrifluorométhylphényl-1 cinnolinone-4
- Amino-3 dihydro-1,4 diméthoxy-3,4 phényl-1 cinnolinone-4
- Amino-3 benzyl-1 dihydro-1,4 cinnolinone-4
- Amino-3 dihydro-1,4 métatrifluorométhylbenzyl-1 cinnolinone-4
- Benzyloxy-3 dihydro-1,4 éthyl-1 cinnolinone-4

3. Procédé de préparation d'un composé selon l'une des revendications 1 et 2 caractérisé en ce que l'on traite un acide dihydro-1,4 cinnolinone-4 carboxylique-3 par le diphényl phosphoryl azide dans un alcool linéaire ou ramifié contenant de 1 à 5 atomes de carbone, à une température comprise entre la température ambiante et la température d'ébullition de l'alcool, puis on traite le produit formé par hydrolyse acide pour obtenir les composés de formule 1 dans lesquels A=NR₃ et R₁, R₃=H.

4. Procédé de préparation d'un composé selon la revendication 3 caractérisé en ce que l'alcool est le tertiobutanol.

5. Procédé de préparation d'un composé selon l'une des revendications 1 et 2 caractérisé en ce que l'on traite une dihydro-1,4 méthylsulfonyl-3 cinnolinone-4 par le formamide dans le diméthylformamide en présence d'une base forte puis par la soude ou la potasse éthanolique pour obtenir les composés de formule I dans lesquels A=NR₃, R₁= R₃ = H.

6. Procédé de préparation d'un composé selon la revendication 5 caractérisé en ce que la base utilisée est l'hydrure de sodium ou de potassium.

7. Procédé de préparation d'un composé selon l'une des revendications 1 et 2 caractérisé en ce que l'on traite une amino-3 dihydro-1,4 cinnolinone-4 par le formaldéhyde dans l'acide formique puis par le cyanoborohydrure de sodium pour obtenir les composés de formule I dans lequels A=NR₃, R₃=R₁=CH₃.

8. Procédé de préparation d'un composé selon l'une des revendications 1 et 2 caractérisé en ce que l'on traite une amino-3 dihydro-1,4 cinnolinone-4 par un nitrite d'alcoyle en présence de chlorure cuivreux en milieu anhydre puis par une amine 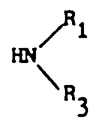 pour obtenir les composés de formule I dans lesquels A=NR₃, R₁ et R₃ sont différents d'hydrogène.

9. Procédé de préparation d'un composé selon la revendication 8 caractérisé en ce que le nitrite d'alcoyle est le nitrite de tertiobutyle et que le milieu anhydre est le diméthylformamide en présence de tamis moléculaires.

10. Procédé de préparation d'un composé selon l'une des revendications 1 et 2 caractérisé en ce que l'on traite une amino-3 dihydro-1,4 cinnolinone-4 par le nitrite de sodium en milieu chlorhydrique pour obtenir les composés de formule I dans lesquels A=oxygène, R₁=H.

11. Procédé de préparation d'un composé selon l'une des revendications 1 et 2 caractérisé en ce que l'on traite une dihydro-1,4 hydroxy-3 cinnolinone-4 telle qu'elle peut être obtenue par le procédé selon la revendication 10 par un dérivé R₁Y, dans lequel R₁ a la même signification que précédemment avec R₁ différent de H et Y représente un groupe labile tel qu'halogène ou mésylate, pour obtenir les composés de formule I dans lesquels R₁ est différent d'hydrogène.

12. Procédé de préparation d'un composé selon la revendication 11 caractérisé en ce que la réaction est effectuée en milieu biphasique en présence d'un catalyseur de transfert de phase.

13. Procédé de préparation d'un composé selon l'une des revendications 11 et 12 caractérisé en ce que le milieu biphasique est constitué d'une phase aqueuse choisie parmi la soude ou la potasse et organique choisie parmi le THF ou l'éther éthylique.

14. Procédé de préparation d'un composé selon l'une des revendications 11, 12 et 13 caractérisé en ce que le catalyseur de transfert de phase est un sel d'ammonium quaternaire, tel que chlorure ou bromure de tétrabutylammonium.

15. A titre de médicaments, utiles par exemple dans le traitement des maladies telles que les bronchopneumopathies obstructives chroniques, l'insuffisance respiratoire, l'emphysème, les pathologies cardiovasculaires relatives à l'insuffisance cardiaque, les composés définis selon l'une des revendications 1 et 2.

16. Compositions pharmaceutiques caractérisées en ce qu'elles contiennent comme principe actif au moins un composé selon l'une des revendications 1 et 2 associé à un support pharmaceutique inerte.

## Patentansprüche

1. Von 4-Cinnolinonen abgeleitete Verbindungen der allgemeinen Formel (I) 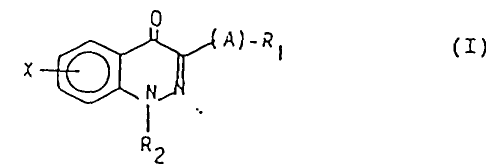 worin bedeuten:
A Sauerstoff oder eine NR₃-Gruppe, worin R₃ Wasserstoff oder ein C₁₋₄-Alkylrest darstellt,
R₁
. Wasserstoff oder einen C₁_₄-Alkylrest
. einen C₁₋₄-Phenylalkylrest, wobei der aromatische Kern substituiert sein kann durch eine oder mehrere Gruppen, ausgewählt aus Halogen, C₁₋₃-Alkyl, C₁₋₃-Alkyloxy und Trifluoromethyl,
. eine Aminoalkylgruppe der Formel 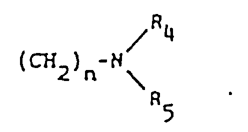 worin n eine ganze Zahl zwischen 1 und einschließlich 4 darstellt und R₄ und R₅, die gleich oder verschieden sind, Wasserstoff oder einen C₁₋₃-Alkylrest darstellen oder gemeinsam zusammen mit dem Stickstoffatom, an das sie gebunden sind, einen Heterocyclus, ausgewählt aus Pyrrolidin, Piperidin, Morpholin oder Piperazin, bilden,
R₂
. einen C₂₋₅-Alkyl-, C₃₋₅-Alkenyl-, C₂₋₅-Hydroxyalkyl-, C₁₋₃-Alkoxy-C₁₋₄-alkyl-Rest,
. einen Phenyl- oder Phenyl-C₁₋₃-alkyl-Rest, wobei der aromatische Kern substituiert sein kann durch eine oder mehr Gruppen, ausgewählt aus Halogen, Cₗ₋₃-Alkyl, C₁₋₃-Alkyloxy und Trifluoromethyl, und
X eine oder mehr Gruppen, ausgewählt aus Wasserstoff, Halogen, C₁₋₃-Alkyl, C₁₋₃-Alkyloxy und Trifluoromethyl.

2. Verbindungen der allgemeinen Formel (I) nach Anspruch 1, dadurch gekennzeichnet, daß sie ausgewählt werden aus der Gruppe:
- 3-Amino-1,4-dihydro-1-ethyl-4-cinnolinon,
- 1,4-Dihydro-3-methylamino-1-metatrifluoromethylphenyl-4-cinnolinon
- 3-Amino-6-chloro-1-ethyl-1,4-dihydro-4-cinnolinon
- 1,4-Dihydro-3-dimethylamino-1-ethyl-4-cinnolinon
- 1,4-Dihydro-1β-metatrifluoromethylphenyl-3-morpholinoethylamino-4-cinnolinon,
- 1,4-Dihydro-1-ethyl-3-hydroxy-4-cinnolinon,
- 1,4-Dihydro-1-ethyl-3-methoxy-4-cinnolinon,
- 1,4-Dihydro-1-ethyl-3-methylamino-4-cinnolinon,
- 3-Benzylamino-1,4-dihydro-1-ethyl-4-cinnolinon,
- 3-Amino-1,4-dihydro-6,7-dimethoxy-1-ethyl-4-cinnolinon,
- 3-Amino-1,4-dihydro-1-ethyl-6,7-methylendioxy-4-cinnolinon,
- 3-Amino-7-chloro-1,4-dihydro-1-ethyl-4-cinnolinon,
- 3-Amino-6-bromo-1,4-dihydro-1-ethyl-4-cinnolinon,
- 3-Amino-1,4-dihydro-1-ethyl-5-methyl-4-cinnolinon,
- 3-Amino-1,4-dihydro-1-ethyl-6-methyl-4-cinnolinon,
- 1-Allyl-3-amino-1,4-dihydro-4-cinnolinon,
- 3-Amino-1,4-dihydro-1-metatrifluoromethylphenyl-4-cinnolinon,
- 1,4-Dihydro-3-dimethylamino-1-metatrifluoromethylphenyl-4-cinnolinon,
- 1,4-Dihydro-3-ethylamino-1-metatrifluoromethylphenyl-4-cinnolinon,
- 3-Benzylamino-1,4-dihydro-1-metatrifluoromethylphenyl-4-cinnolinon,
- 3-Amino-1,4-dihydro-3,4-dimethoxy-1-phenyl-4-cinnolinon,
- 3-Amino-1-benzyl-1,4-dihydro-4-cinnolinon,
- 3-Amino-1,4-dihydro-1-metatrifluoromethylbenzyl-4-cinnolinon,
- 3-Benzyloxy-1,4-dihydro-1-ethyl-4-cinnolinon.

3. Verfahren zur Herstellung einer Verbindung nach einem der Ansprüche 1 und 2, dadurch gekennzeichnet, daß man eine 1,4-Dihydro-4-cinnolinon-3-carbonsäure mit Diphenylphosphorylazid in einem linearen oder verzweigten Alkohol, der 1 bis 5 Kohlenstoffatome enthält, bei einer Temperatur zwischen der Umgebungstemperatur und der Siedetemperatur des Alkohols behandelt, dann das gebildete Produkt einer sauren Hydrolyse unterwirft, wobei man die Verbindungen der Formel (I) erhält, worin A = NR₃ und R₁, R₃ = H.

4. Verfahren zur Herstellung einer Verbindung nach Anspruch 3, dadurch gekennzeichnet, daß der Alkohol tert-Butanol ist.

5. Verfahren zur Herstellung einer Verbindung nach einem der Ansprüche 1 und 2, dadurch gekennzeichnet, daß man ein 1,4-Dihydro-3-methylsulfonyl-4-cinnolinon mit Formamid in Dimethylformamid in Gegenwart einer starken Base und danach mit ethanolischem Natriumhydroxid oder Kaliumhydroxid behandelt, wobei man die Verbindungen der Formel (I) erhält, worin A = NR₃, R₁ = R₃ = H.

6. Verfahren zur Herstellung einer Verbindung nach Anspruch 5, dadurch gekennzeichnet, daß die verwendete Base Natrium- oder Kaliumhydrid ist.

7. Verfahren zur Herstellung einer Verbindung nach einem der Ansprüche 1 und 2, dadurch gekennzeichnet, daß man ein 3-Amino-1,4-dihydro-4-cinnolinon mit Formaldehyd in Ameisensäure und danach mit Natriumcyanoborhydrid behandelt, wobei man die Verbindungen der Formel (I) erhält, worin A = NR₃, R₃ = R₁ = CH₃.

8. Verfahren zur Herstellung einer Verbindung nach einem der Ansprüche 1 und 2, dadurch gekennzeichnet, daß man ein 3-Amino-1,4-dihydro-4-cinnolinon mit einem Alkylnitrit in Gegenwart von Kupfer(I)chlorid in einem wasserfreien Medium und danach mit einem Amin der Formel 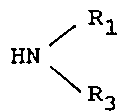 behandelt, wobei man die Verbindungen der Formel (I) erhält, worin A = NR₃, R₁ und R₃ von Wasserstoff verschieden sind.

9. Verfahren zur Herstellung einer Verbindung nach Anspruch 8, dadurch gekennzeichnet, daß das Alkylnitrit tert-Butylnitrit ist und daß das wasserfreie Medium Dimethylformamid in Gegenwart von Molekularsieben ist.

10. Verfahren zur Herstellung einer Verbindung nach einem der Ansprüche 1 und 2, dadurch gekennzeichnet, daß man ein 3-Amino-1,4-dihydro-4-cinnolinon in einem Chlorwasserstoffsäure-Medium mit Natriumnitrit behandelt, wobei man die Verbindungen der Formel (I) erhält, worin A = Sauerstoff und R₁ = H.

11. Verfahren zur Herstellung einer Verbindung nach einem der Ansprüche 1 und 2, dadurch gekennzeichnet, daß man ein 1,4-Dihydro-3-hydroxy-4-cinnolinon, wie es bei dem Verfahren gemäß Anspruch 10 erhalten werden kann, mit einem R₁Y-Derivat behandelt, worin R₁ die gleiche Bedeutung wie oben hat, die jedoch von H verschieden ist, und Y eine labile Gruppe wie Halogen oder Mesylat darstellt, wobei man die Verbindungen der Formel (I) erhält, worin R₁ von Wasserstoff verschieden ist.

12. Verfahren zur Herstellung einer Verbindung nach Anspruch 11, dadurch gekennzeichnet, daß die Reaktion in einem biphasischen Medium in Gegenwart eines Phasenübertragungskatalysators durchgeführt wird.

13. Verfahren zur Herstellung einer Verbindung nach einem der Ansprüche 11 und 12, dadurch gekennzeichnet, daß das biphasische Medium besteht aus einer wäßrigen Phase, ausgewählt aus Natronlauge (Natriumhydroxid) oder Kalilauge (Kaliumhydroxid) und einer organischen Phase, ausgewählt aus THF oder Ethyläther.

14. Verfahren zur Herstellung einer Verbindung nach einem der Ansprüche 11, 12 und 13, dadurch gekennzeichnet, daß der Phasenübertragungskatalysator ein quaternäres Ammoniumsalz wie Tetrabutylammoniumchlorid oder -bromid ist.

15. Verwendung der Verbindungen nach einem der Ansprüche 1 und 2 als Arzneimittel, die beispielsweise für die Behandlung von Erkrankungen, wie Bronchopneumopathien, chronischen Erkrankungen, Atmungsinsuffizienz, Emphysem und Cardiovasculärerkrankungen im Zusammenhang mit Herzinsuffizienz, verwendbar sind.

16. Pharmazeutische Zusammensetzungen, dadurch gekennzeichnet, daß sie als aktives Prinzip (Wirkstoff) mindestens eine Verbindung nach einem der Ansprüche 1 und 2 in Kombination mit einem inerten pharmazeutischen Träger enthalten.

## Claims

1. Compounds derived from 4-cinnolinones of general formula I 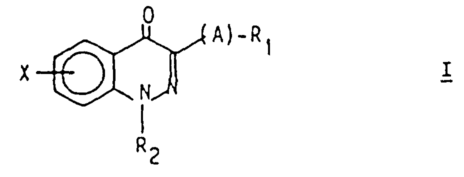 in which:
- A represents oxygen or a group NR₃ in which R₃ itself represents hydrogen or a C₁₋₄ alkyl radical
- R₁ represents:
. hydrogen or a C₁₋₄ alkyl radical
. a C₁₋₄ phenylalkyl radical, it being possible for the aromatic nucleus to be substituted with one or more groups chosen from: halogen, C₁₋₃alkyl, C₁₋₃alkyloxy or trifluoromethyl,
. an aminoalkyl group 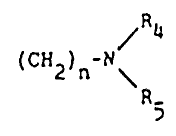 in which n represents an integer between 1 and 4 inclusive and R₄ and R₅, which may be identical or different, represent hydrogen or a C₁₋₃alkyl radical, or together form, with the nitrogen atom to which they are attached, a heterocycle such as pyrrolidine, piperidine, morpholine or piperazine.
- R₂ represents:
. a C₂₋₅ alkyl, C₃₋₅ alkenyl, C₂₋₅ hydroxyalkyl, C₁₋₃ alkoxy C₁₋₄ alkyl radical
. a phenyl or C₁₋₃ alkyl phenyl radical, it being possible for the aromatic nucleus to be substituted with one or more groups chosen from halogen, C₁₋₃ alkyl, C₁₋₃ alkyloxy or trifluoromethyl.
- X represents one or more groups such as hydrogen, halogen, C₁₋₃ alkyl, C₁₋₃ alkyloxy or trifluoromethyl.

2. Compounds of general formula I according to Claim 1, characterized in that they are chosen from:
- 3-Amino-1,4-dihydro-1-ethyl-4-cinnolinone
- 1,4-Dihydro-3-methylamino-1-metatrifluoromethylphenyl-4-cinnolinone
- 3-Amino-6-chloro-1-ethyl-1,4-dihydro-4-cinnolinone
- 1,4-Dihydro-3-dimethylamino-1-ethyl-4-cinnolinone
- 1,4-Dihydro-1-metatrifluoromethylphenyl-3-β-morpholinoethylamino-4-cinnolinone
- 1,4-Dihydro-1-ethyl-3-hydroxy-4-cinnolinone
- 1,4-Dihydro-1-ethyl-3-methoxy-4-cinnolinone
- 1,4-Dihydro-1-ethyl-3-methylamino-4-cinnolinone
- 3-Benzylamino-1,4-dihydro-1-ethyl-4-cinnolinone
- 3-Amino-1,4-dihydro-6,7-dimethoxy-1-ethyl-4-cinnolinone
- 3-Amino-1,4-dihydro-1-ethyl-6,7-methylenedioxy-4-cinnolinone
- 3-Amino-7-chloro-1,4-dihydro-1-ethyl-4-cinnolinone
- 3-Amino-6-bromo-1,4-dihydro-1-ethyl-4-cinnolinone
- 3-Amino-1,4-dihydro-1-ethyl-5-methyl-4-cinnolinone
- 3-Amino-1,4-dihydro-1-ethyl-6-methyl-4-cinnolinone
- 1-Allyl-3-amino-1,4-dihydro-4-cinnolinone
- 3-Amino-1,4-dihydro-1-metatrifluoromethylphenyl-4-cinnolinone
- 1,4-Dihydro-3-dimethylamino-1-metatrifluoromethylphenyl-4-cinnolinone,
- 1,4-Dihydro-3-ethylamino-1-metatrifluoromethylphenyl-4-cinnolinone
- 3-Benzylamino-1,4-dihydro-1-metatrifluoromethylphenyl-4-cinnolinone
- 3-Amino-1,4-dihydro-3,4-dimethoxy-1-phenyl-4-cinnolinone
- 3-Amino-1-benzyl-1,4-dihydro-4-cinnolinone
- 3-Amino-1,4-dihydro-1-metatrifluoromethylbenzyl-4-cinnolinone
- 3-Benzyloxy-1,4-dihydro-1-ethyl-4-cinnolinone

3. Method for preparing a compound according to either of Claims 1 and 2, characterized in that a 1,4-dihydro-4-cinnolinone-3-carboxylic acid is treated with diphenylphosphoryl azide in a linear or branched alcohol containing from 1 to 5 carbon atoms, at a temperature between room temperature and the boiling point of the alcohol, and the product formed is then treated with acid hydrolysis in order to obtain the compounds of formula I in which A = NR₃ and R₁ and R₃ = H.

4. Method for preparing a compound according to Claim 3, characterized in that the alcohol is tert-butanol.

5. Method for preparing a compound according to either of Claims 1 and 2, characterized in that a 1,4 dihydro-3-methylsulphonyl-4-cinnolinone is treated with formamide in dimethylformamide in the presence of a strong base, followed by ethanolic sodium hydroxide or potassium hydroxide, in order to obtain the compounds of formula I in which A = NR₃ and R₁ = R₃ = H.

6. Method for preparing a compound according to Claim 5, characterized in that the base used is sodium hydride or potassium hydride.

7. Method for preparing a compound according to either of Claims 1 and 2, characterized in that a 3-amino-1,4-dihydro-4-cinnolinone is treated with formaldehyde in formic acid, followed by sodium cyanoborohydride, in order to obtain the compounds of formula I in which A = NR₃ and R₃ = R₁ = CH₃.

8. Method for preparing a compound according to either of Claims 1 and 2, characterized in that a 3-amino-1,4-dihydro-4-cinnolinone is treated with an alkyl nitrite in the presence of cuprous chloride in an anhydrous medium, followed by an amine 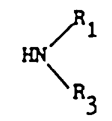 in order to obtain the compounds of formula I in which A = NR₃ and R₁ and R₃ are other than hydrogen.

9. Method for preparing a compound according to Claim 8, characterized in that the alkyl nitrite is tert-butyl nitrite and in that the anhydrous medium is dimethylformamide in the presence of molecular sieves.

10. Method for preparing a compound according to etiher of Claims 1 and 2, characterized in that a 3-amino-1,4-dihydro-4-cinnolinone is treated with sodium nitrite in a hydrochloric medium in order to obtain the compounds of formula I in which A = oxygen and R₁ = H.

11. Method for preparing a compound according to either of Claims 1 and 2, characterized in that a 1,4-dihydro-3-hydroxy-4-cinnolinone as can be obtained by the method according to Claim 10 is treated with a derivative R₁Y, in which R₁ has the same meaning as above and Y represents a labile group such as halogen or mesylate, in order to obtain the compounds of formula I, in which R₁ is other than hydrogen.

12. Method for preparing a compound according to Claim 11, characterized in that the reaction is carried out in a two-phase medium in the presence of a phase transfer catalyst.

13. Method for preparing a compound according to either of Claims 11 and 12, characterized in that the two-phase medium consists of an aqueous phase such as sodium hydroxide or potassium hydroxide and an organic phase such as THF or ethyl ether.

14. Method for preparing a compound according to one of Claims 11, 12 and 13, characterized in that the phase transfer catalyst is a quaternary ammonium salt such as tetrabutylammonium chloride or bromide.

15. By way of medicaments which are useful, for example, for treating diseases such as chronic obstructive bronchopneumopathies, respiratory insufficiency, emphysema and cardiovascular pathologies related to cardiac insufficiency, the compounds defined according to either of Claims 1 and 2.

16. Pharmaceutical compositions, characterized in that they contain as active principle at least one compound according to either of Claims 1 and 2, combined with an inert pharmaceutical vehicle.
